# EUROPEAN PATENT APPLICATION

(11) **EP 4 603 831 A1**
(43) Date of publication of application: **20.08.2025**
(21) Application number: 22962139.6
(22) Date of filing: 11.10.2022
(51) Int. Cl.: G01N 27/30, G01N 27/327

(54) **BIOSENSOR**

(71) Applicant: LG Electronics Inc., Yeongdeungpo-gu Seoul 07336 (KR)
(72) Inventor: MOON, Jinsan, Seoul 06772 (KR); SUNG, Jinwoo, Seoul 06772 (KR)
(74) Representative: Schott, Jakob Valentin
(86) International application number: PCT/KR2022/015247
(87) International publication number: WO 2024/080388

(57) **Abstract**

The present disclosure relates to a biosensor. The biosensor comprises a substrate, a graphene layer disposed on at least one concave pattern formed on the substrate, a source electrode formed on one end of the graphene layer and the substrate, a drain electrode separated from the source electrode and formed on the other end of the graphene layer and the substrate, and a first passivation layer and a second passivation layer respectively disposed on the source electrode and the drain electrode. Accordingly, sensing sensitivity of a graphene-based sensor can be improved.

## Description

### BACKGROUND

### 1. Field of the disclosure

The present disclosure relates to a biosensor and, more specifically, to a biosensor capable of improving the sensing sensitivity of a graphene-based sensor.

### 2. Description of the Related Art

Recently, as diseases having a high infectivity applied, a need for rapid diagnosis and self-diagnosis of the disease in medical fields such as homes, hospitals, and public health centers is increasing.

Therefore, it is required to develop an immunoassay platform with short analysis time and high accuracy without requiring specialized knowledge or complicated procedures.

A biosensor generates an electrical, optical signal, and a color that changes according to a selective reaction between probe material having reactivity for a specific target material contained in a body fluid such as sweat and saliva, or in biological substances such as blood or urine, and the target material. Accordingly, it is possible to check the presence of a specific target material by using the biosensor.

Meanwhile, methods using graphene materials are being studied for the fabrication of biosensors.

A paper entitled "Digital Biosensing by Foundry-Fabricated Graphene Sensors," published in Scientific Reports on January 22, 2009 (hereinafter, it is referred to as 'prior literature'), discloses a method of disposing graphene on a substrate.

However, the prior literature reveals a disadvantage in that electrostatic gating is not performed over the entire graphene channel due to the lattice mismatch between a substrate and a graphene layer, which is caused by a passivation layer covering a part of the graphene layer, and thus the sensing sensitivity is lowered.

### SUMMARY

An object of the present disclosure is to provide a biosensor capable of improving the sensing sensitivity of a graphene-based sensor.

Meanwhile, another object of the present disclosure is to provide a biosensor in which at least a portion of the lower surface of the graphene layer is separated from the substrate.

To achieve the objects above, a biosensor according to one embodiment of the present disclosure comprises a substrate, a graphene layer disposed on at least one concave pattern formed on the substrate, a source electrode formed on one end of the graphene layer and the substrate, a drain electrode separated from the source electrode and formed on the other end of the graphene layer and the substrate, and a first passivation layer and a second passivation layer respectively disposed on the source electrode and the drain electrode.

Meanwhile, a portion of the first passivation layer can be formed beneath one end of the graphene layer, and a portion of the second passivation layer can be formed beneath the other end of the graphene layer.

Meanwhile, the graphene layer can be disposed on a plurality of concave patterns formed on the substrate.

Meanwhile, the graphene layer can be disposed on a plurality of concave patterns formed on the substrate, and a plurality of convex patterns can be formed on the graphene layer, the plurality of convex patterns being adjacent to the plurality of concave patterns.

Meanwhile, the plurality of convex patterns can be formed of the same material as the first passivation layer and the second passivation layer.

Meanwhile, the bio sensor can further include a gate electrode separated from the upper portion of either the first passivation layer or the second passivation layer.

Meanwhile, a portion of the graphene layer can be covered by the source electrode or the drain electrode and the first passivation layer or the second passivation layer; and the other portion of the graphene layer can be left open without being covered by the source electrode or the drain electrode and the first passivation layer or the second passivation layer.

Meanwhile, the source electrode can be formed on a first area which includes the one end of the graphene layer; the first passivation layer can be formed on a second area adjacent to the first area of the graphene layer; the passivation layer cannot be covered on a third area adjacent to the second area of the graphene layer; the second passivation layer can be formed on a fourth area adjacent to the third area of the graphene layer; and the drain electrode can be formed on a fifth area which includes the other end of the graphene layer and is adjacent to the fourth area.

Meanwhile, the length of the second area or the fourth area of the graphene layer is preferably shorter than the length of the third area.

Meanwhile, the length of the second area or the fourth area of the graphene layer can be 100 nm or less.

Meanwhile, the source electrode or the drain electrode can include an organic compound or an inorganic compound, and the inorganic compound can include at least one of WO₃, MoO₃, or ZnO.

Meanwhile, a biosensor according to one embodiment of the present disclosure can further include an insulating layer disposed on the substrate; and the source electrode, the drain electrode, and the graphene layer can be disposed on the insulating layer.

To achieve the objects above, a biosensor according to another embodiment of the present disclosure comprises a substrate; a graphene layer formed on the substrate; a source electrode formed on one end of the graphene layer and the substrate; a drain electrode separated from the source electrode and formed on the other end of the graphene layer and the substrate; and a first passivation layer and a second passivation layer disposed on the source electrode and the drain electrode, respectively, wherein at least a portion of the lower surface of the graphene layer is separated from the substrate.

Meanwhile, at least one concave pattern can be formed in a partial area of the lower surface of the graphene layer within the substrate.

Meanwhile, a plurality of concave patterns can be formed in a partial area of the lower surface of the graphene layer within the substrate.

Meanwhile, a plurality of concave patterns can be formed in a partial area of the lower surface of the graphene layer within the substrate, and a plurality of convex patterns can be formed in a partial area of the upper surface of the graphene layer.

### EFFECTS OF THE DISCLOSURE

A biosensor according to one embodiment of the present disclosure comprises a substrate, a graphene layer disposed on at least one concave pattern formed on the substrate, a source electrode formed on one end of the graphene layer and the substrate, a drain electrode separated from the source electrode and formed on the other end of the graphene layer and the substrate, and a first passivation layer and a second passivation layer respectively disposed on the source electrode and the drain electrode. Accordingly, it is possible to improve the sensing sensitivity of a graphene-based sensor. Also, at least a portion of the lower surface of the graphene layer can be separated from the substrate.

Meanwhile, a portion of the first passivation layer can be formed beneath one end of the graphene layer, and a portion of the second passivation layer can be formed beneath the other end of the graphene layer. Accordingly, it is possible to improve the sensing sensitivity of a graphene-based sensor.

Meanwhile, the graphene layer can be disposed on a plurality of concave patterns formed on the substrate. Accordingly, it is possible to improve the sensing sensitivity of a graphene-based sensor.

Meanwhile, the graphene layer can be disposed on a plurality of concave patterns formed on the substrate, and a plurality of convex patterns can be formed on the graphene layer, the plurality of convex patterns being adjacent to the plurality of concave patterns. Accordingly, it is possible to improve the sensing sensitivity of a graphene-based sensor.

Meanwhile, the plurality of convex patterns can be formed of the same material as the first passivation layer and the second passivation layer. Accordingly, it is possible to improve the sensing sensitivity of a graphene-based sensor.

Meanwhile, the bio sensor can further include a gate electrode separated from the upper portion of either the first passivation layer or the second passivation layer. Accordingly, it is possible to improve the sensing sensitivity of a graphene-based sensor.

Meanwhile, a portion of the graphene layer can be covered by the source electrode or the drain electrode and the first passivation layer or the second passivation layer; and the other portion of the graphene layer can be left open without being covered by the source electrode or the drain electrode and the first passivation layer or the second passivation layer. Accordingly, it is possible to improve the sensing sensitivity of a graphene-based sensor.

Meanwhile, the source electrode can be formed on a first area which includes the one end of the graphene layer; the first passivation layer can be formed on a second area adjacent to the first area of the graphene layer; the passivation layer cannot be covered on a third area adjacent to the second area of the graphene layer; the second passivation layer can be formed on a fourth area adjacent to the third area of the graphene layer; and the drain electrode can be formed on a fifth area which includes the other end of the graphene layer and is adjacent to the fourth area. Accordingly, it is possible to improve the sensing sensitivity of a graphene-based sensor.

Meanwhile, the length of the second area or the fourth area of the graphene layer is preferably shorter than the length of the third area. Accordingly, it is possible to improve the sensing sensitivity of a graphene-based sensor.

Meanwhile, the length of the second area or the fourth area of the graphene layer can be 100 nm or less. Accordingly, it is possible to improve the sensing sensitivity of a graphene-based sensor.

Meanwhile, the source electrode or the drain electrode can include an organic compound or an inorganic compound, and the inorganic compound can include at least one of WO₃, MoO₃, or ZnO. Accordingly, it is possible to improve the sensing sensitivity of a graphene-based sensor.

Meanwhile, a biosensor according to one embodiment of the present disclosure can further include an insulating layer disposed on the substrate; and the source electrode, the drain electrode, and the graphene layer can be disposed on the insulating layer. Accordingly, it is possible to improve the sensing sensitivity of a graphene-based sensor.

To achieve the objects above, a biosensor according to another embodiment of the present disclosure comprises a substrate; a graphene layer formed on the substrate; a source electrode formed on one end of the graphene layer and the substrate; a drain electrode separated from the source electrode and formed on the other end of the graphene layer and the substrate; and a first passivation layer and a second passivation layer disposed on the source electrode and the drain electrode, respectively, wherein at least a portion of the lower surface of the graphene layer is separated from the substrate. Accordingly, it is possible to improve the sensing sensitivity of a graphene-based sensor.

Meanwhile, at least one concave pattern can be formed in a partial area of the lower surface of the graphene layer within the substrate. Accordingly, it is possible to improve the sensing sensitivity of a graphene-based sensor.

Meanwhile, a plurality of concave patterns can be formed in a partial area of the lower surface of the graphene layer within the substrate. Accordingly, it is possible to improve the sensing sensitivity of a graphene-based sensor.

Meanwhile, a plurality of concave patterns can be formed in a partial area of the lower surface of the graphene layer within the substrate, and a plurality of convex patterns can be formed in a partial area of the upper surface of the graphene layer. Accordingly, it is possible to improve the sensing sensitivity of a graphene-based sensor.

### BRIEF DESCRIPTION OF THE DRAWINGS

FIG. 1 is one example of a side view of a biosensor according to one embodiment of the present disclosure.
FIG. 2A is a simplified perspective view of the biosensor of FIG. 1.
FIG. 2B is one example of a top view of the biosensor of FIG. 1.
FIG. 3 is one example of a side view of a biosensor related to the present disclosure.
FIG. 4 is a diagram referred to in the description of a biosensor according to one embodiment of the present disclosure of FIG. 1.
FIGS. 5 to 7B are side views of biosensors according to various embodiments of the present disclosure.

### DETAILED DESCRIPTION OF THE PREFERRED EMBODIMENTS

In what follows, the present disclosure will be described in more detail with reference to appended drawings.

The suffixes "module" and "unit" for the constituting elements used in the following descriptions are assigned only for the convenience of writing the present disclosure and do not have separate meanings or roles distinguished from each other. Therefore, the "module" and "unit" can be used interchangeably.

In the present specification, target materials are biomaterials representing a specific substrate, and are interpreted as having the same meaning as analytical bodies or analytes. In the present embodiment, the target material can be an antigen. In the present specification, probe material is a biomaterial that specifically binds to a target material and is interpreted as having the same meaning as a receptor or an acceptor. In the present embodiment, the probe material can be an antibody.

The electrochemical-based biosensor combines the analytical ability of the electrochemical method with a specificity of biological recognition and detects a biological recognition phenomenon for a target material as a change in current or potential, by immobilizing or containing a material having biological specificity, i.e., probe material such as an enzyme, an antigen, an antibody, or a biochemical material, on the surface of an electrode.

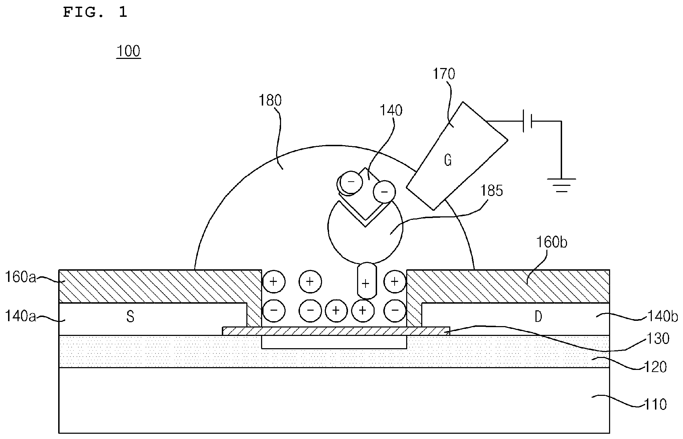

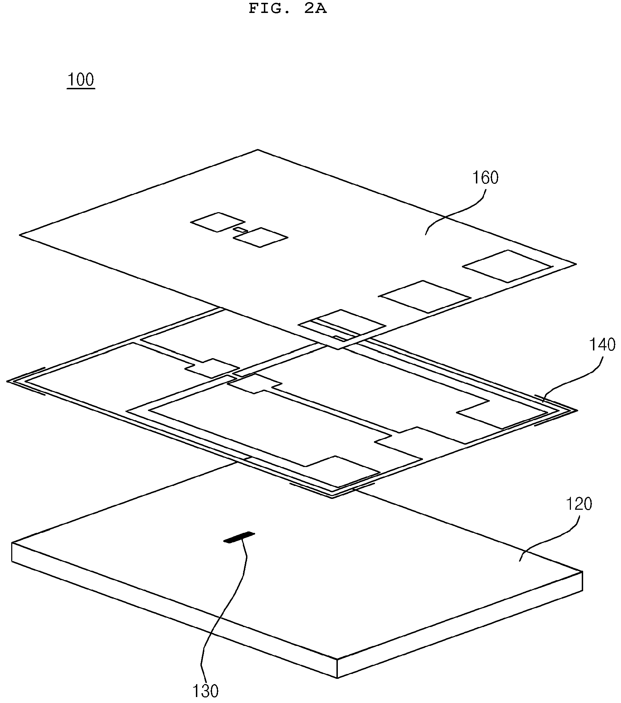

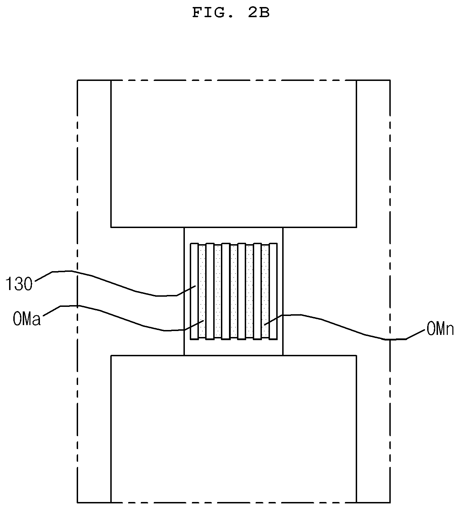

FIG. 1 is one example of a side view of a biosensor according to one embodiment of the present disclosure, FIG. 2A is a simplified perspective view of the biosensor of FIG. 1, and FIG. 2B is one example of a top view of the biosensor of FIG. 1.

Referring to the figure, the biosensor 100 according to one embodiment of the present disclosure includes a graphene-based field effect transistor (FET) using the graphene layer 130 as a channel.

To this end, the biosensor 100 according to one embodiment of the present disclosure comprises a substrate 110; a graphene layer 130 disposed on at least one concave pattern formed on the substrate 110 or an insulating layer 120, a source electrode 140a formed on one end of the graphene layer 130 and the substrate 110, a drain electrode 140b separated from the source electrode 140a and formed on the other end of the graphene layer 130 and the substrate 110, and a first passivation layer 160a and a second passivation layer 160b respectively disposed on the source electrode 140a and the drain electrode 140b.

According to the biosensor 100 of one embodiment of the present disclosure, at least a portion of the lower surface of the graphene layer 130 is separated from the substrate 110. Accordingly, a lattice mismatch between the substrate 110 and the graphene layer 130 is significantly reduced, eventually improving graphene-based sensing sensitivity.

Meanwhile, the biosensor 100 according to one embodiment of the present disclosure can further include a gate electrode 170 disposed on top of and separated from either of the first passivation layer 160a and the second passivation layer 160b. Accordingly, it is possible to improve the sensing sensitivity of a graphene-based sensor.

Meanwhile, the biosensor 100 according to one embodiment of the present disclosure further includes an insulating layer 120 disposed on the substrate 110.

In other words, the source electrode 140a, the drain electrode 140b, and the graphene layer 130 can be disposed on the insulating layer 120. Accordingly, it is possible to improve the sensing sensitivity of a graphene-based sensor.

Meanwhile, a biosensor 100 according to another embodiment of the present disclosure comprises a substrate 110; an insulating layer 120 on the substrate; a graphene layer 130 formed on the insulating layer 120; a source electrode 140a formed on one end of the graphene layer 130 and the substrate 110; a drain electrode 140b separated from the source electrode 140a and formed on the other end of the graphene layer 130 and the substrate 110; and a first passivation layer 160a and a second passivation layer 160b disposed on the source electrode 140a and the drain electrode 140b, respectively, wherein at least a portion of the lower surface of the graphene layer 130 is separated from the substrate 110. Accordingly, it is possible to improve the sensing sensitivity of a graphene-based sensor.

Meanwhile, the substrate 110 is a semiconductor substrate, which can be made up of a silicon substrate.

Meanwhile, the insulating layer 120 on the substrate 110 can be formed of silicon oxide (SiO2) or silicon nitride. For example, a silicon oxide-based insulating layer 120 can be formed on the surface through heat treatment.

Meanwhile, at least one concave pattern OM can be formed in a partial area of the insulating layer 120. For example, as illustrated in FIG. 2B, a plurality of concave patterns OMa to OMn can be formed in a stripe shape.

In this case, the concave pattern OM can be an etching pattern or a printing pattern formed by a photoresist, an etching solution, or the like.

Alternatively, the concave pattern OM can be a sputter pattern formed by a sputtering process (not shown in the figure).

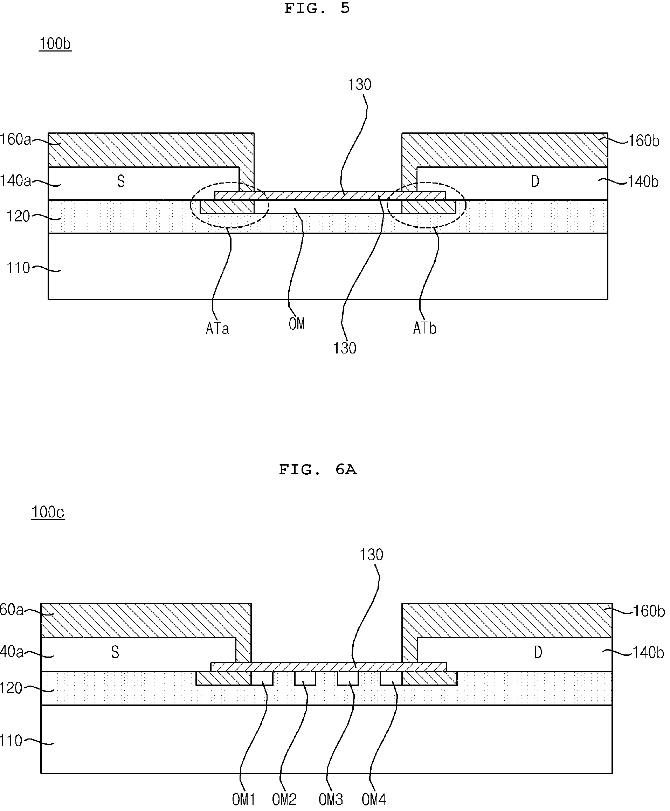

Although a single concave pattern OM is illustrated in the figure, a plurality of concave patterns can also be employed. Meanwhile, the plurality of concave patterns can be formed in various shapes, such as stripe, mesh, hexagonal, or dot patterns. The plurality of concave patterns will be further described with reference to FIG. 6A and subsequent figures.

Meanwhile, a graphene layer 130 is formed on the insulating layer 120, on which at least one concave pattern OM is formed.

The graphene layer 130 is partially exposed for sensing, while another area is covered by the passivation layers 160a and 160b, and yet another area is connected to the source electrode 140a or the drain electrode 140b.

Meanwhile, the graphene layer 130 can be formed in plurality in the biosensor 100.

Meanwhile, the source electrode 140a and the drain electrode 140b are spaced apart from each other and formed on a portion of the insulating layer 120 and the graphene layer 130.

The figure illustrates a case in which the source electrode 140a is formed on the left area of the insulating layer 120 and one end of the graphene layer 130, and the drain electrode 140b is formed on the right area of the insulating layer 120 and the other end of the graphene layer 130.

The source electrode 140a and the drain electrode 140b can be formed into the same layer. Accordingly, the source electrode 140a and the drain electrode 140b can be formed into the same layer through the same process.

For example, the source electrode 140a and the drain electrode 140b can be formed respectively by forming electrode layers and patterning the corresponding electrode layers simultaneously.

Meanwhile, in addition to the source electrode 140a and the drain electrode 140b, the gate electrode 170 can also be formed into the same layer through the same process.

As described above, by simultaneously forming the source electrode 140a, the drain electrode 140b, and the gate electrode 170 that do not overlap with each other, it is possible to reduce the number of process steps and process time and cost.

The source electrode 140a, the drain electrode 140b, and the gate electrode 170 can include at least one of, but not limited to, Ni, Zn, Pd, Ag, Cd, Pt, Ga, In, and Au.

Meanwhile, the gate electrode 170 is formed being separated from the source electrode 140a and the drain electrode 140b.

In particular, as shown in the figure, the gate electrode 170 can be disposed on top of and separated from either of the first passivation layer 160a and the second passivation layer 160b.

The gate electrode 170 can have a larger area than the source electrode 140a and the drain electrode 140b.

Meanwhile, the source electrode 150a or the drain electrode 150b can include an organic compound or an inorganic compound, wherein the inorganic compound can include at least one of WO₃, MoO₃, or ZnO. Accordingly, the sensing sensitivity of the graphene-based sensor can be improved.

Next, the first passivation layer 160a and the second passivation layer 160b are covered onto the source electrode 150a and the drain electrode 150b, respectively.

Meanwhile, the source electrode 150a and the first passivation layer 160a are covered onto the left portion of the graphene layer 130, while the drain electrode 150b and the second passivation layer 160b are covered onto the right portion of the graphene layer 130.

The central portion of the graphene layer 130 is exposed to the outside without being covered by the source electrode 150a, the drain electrode 150b, the first passivation layer 160a, or the second passivation layer 160b.

The passivation layers 160a and 160b can be formed of a moisture-resistant material to protect the source electrode 140a and the drain electrode 140b.

In one example, the passivation layers 160a and 160b can be formed of an oxide layer, a nitride layer, or a carbide layer.

In another example, the passivation layers 160a and 160b can include at least one of inorganic materials such as SiO₂, SixNx, Al₂O₃, or TiO₂, or polymer materials such as epoxy-based SU or polyimide-based polymer.

Also, the passivation layers 160a, 160b can be covered as, but are not limited to, polymer resin.

Referring to the figure, since at least a portion of the lower surface of the graphene layer 130 is separated from the substrate 110 due to at least one concave pattern OM formed on the substrate 110, the lattice mismatch between the substrate 110 and the graphene layer 130 is significantly reduced, eventually improving the graphene-based sensing sensitivity.

At this time, as the separation area between the lower surface of the graphene layer 130 and the substrate 110 increases, the graphene-based sensing sensitivity can be further improved.

Meanwhile, a specimen solution 180 can contact an open area of the graphene layer 130 and a portion of the gate electrode 170 of the biosensor 100 according to the present embodiment.

The specimen solution 180 is an ionic solution and includes a sensing material 185 that reacts explicitly to a target material 140 to be sensed by the biosensor 100.

For example, when the target material 140 is an antigen, an antibody can be attached to the sensing material 185, and when the target material 140 is an antibody, an antigen can be attached to the sensing material 185.

Meanwhile, a linker material (not shown in the figure) can be attached for a smooth connection between the sensing material 185 and the graphene layer 130. The linker material can be different depending on the graphene layer 130 and the sensing material 185.

When the graphene layer 130 is a polymer structure in the nanoscale, the linker material can be formed of at least one of polyurethane, polydimethylsiloxane, Norland Optical Adhesives (NOA), epoxy, polyethylene terephthalate, polymethyl methacrylate, polyimide, polystyrene, polyethylene naphthalate, polycarbonate, or a combination thereof.

In addition, the linker material can be formed of a combination of polyurethane and NOA (e.g. NOA 68). However, the linker material is not limited thereto, and can be made of various polymers having flexibility.

Meanwhile, when the target material 140 is present in the specimen solution 180 while the specimen solution 180 is applied, and relevant voltages are introduced to the source electrode 140a, the drain electrode 140b, and the gate electrode 170, respectively, the target material 140 interacts with a sensing material 185, which charges the graphene layer 130 with specific carriers. Accordingly, a depletion state in which charges are accumulated in the graphene layer 130 proceeds, and a drain current flowing through the drain electrode 140b increases.

Here, the number of accumulated charges can be proportional to the area of the graphene layer 130. Accordingly, when there are a plurality of graphene layers 130, the drain current flowing through the drain electrode 140b is amplified.

Meanwhile, when the target material 140 does not exist in the specimen solution 180 while the specimen solution 180 is applied, and relevant voltages are introduced to the source electrode 140a, the drain electrode 140b, and the gate electrode 170, respectively, the drain current flows through the drain electrode 140b at a significantly lower level than the drain current when the target material 140 exists.

Meanwhile, the specimen solution 180 can refer to a biological material, such as a solution diluted by saliva, a body fluid including sweat, blood, serum, or plasma.

Meanwhile, the biosensor 100 can have various sizes depending on the type of target material, the number of target materials, and the size of the cartridge 100; for example, the biosensor 100 can be designed to have a size of 6*6mm or 6*8mm.

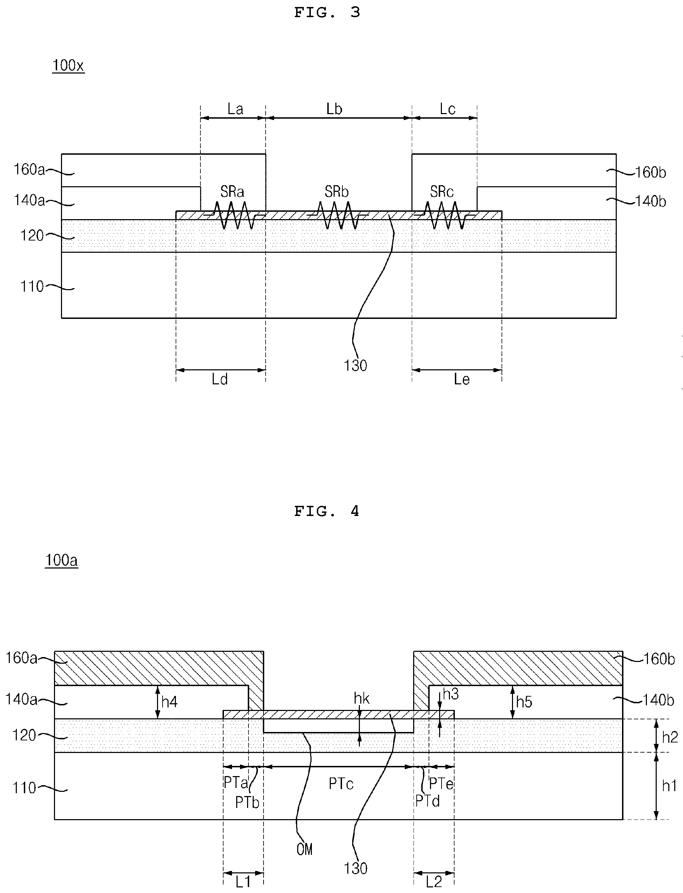

FIG. 3 is one example of a side view of a biosensor related to the present disclosure.

Referring to the figure, the biosensor 100x related to the present disclosure comprises a substrate 110, an insulating layer 120 on the substrate 110, a graphene layer 130 disposed on a portion of the insulating layer 120, a source electrode 140a disposed on one end of the graphene layer 130 and on the insulating layer 120, a drain electrode 140b disposed on the other end of the graphene layer 130 and on the insulating layer 120, a first passivation layer 160a disposed on the source electrode 140a, and a second passivation layer 160b disposed on the drain electrode 140b.

According to the structure of FIG. 3, the passivation layers 160a, 160b, the graphene layer 130, and the insulating layer 120 contact in some areas La, Lb.

The figure illustrates a case in which a portion La in the left part Ld of the graphene layer 130 is covered by the first passivation layer 160a, and a portion Lc in the right part Le of the graphene layer 130 is covered by the second passivation layer 160b.

Accordingly, only the central part Lb of the graphene layer 130 is subjected to resistance modulation; however, the portion La of the graphene layer 130 covered by the first passivation layer 160a and the portion Lc of the graphene layer 130 covered by the second passivation layer are not subjected to resistance modulation.

Therefore, the total resistance of the graphene layer 130 is obtained by a sum of the resistance component SRa of a portion La of the graphene layer 130, the resistance component SRc of a portion Lb of the graphene layer 130, and the resistance component SRb of the central portion Lb of the graphene layer 130.

For example, when the resistance component SRa of the portion La of the graphene layer 130 and the resistance component SRc of the portion Lb of the graphene layer 130 are of low resistance, all the resistance components are reflected through channel modulation even if the resistance component SRb of the central portion Lb of the graphene layer 130 is converted from low resistance to high resistance.

Specifically, when the resistance component SRa of the portion La of the graphene layer 130 and the resistance component SRc of the portion Lb of the graphene layer 130 are 1 KΩ, respectively, and the resistance component SRb of the central portion Lb of the graphene layer 130 ranges from 1 to 10 KΩ, the total resistance of the graphene layer 130 is converted to a value between approximately 3KΩ and 12kΩ. Accordingly, the sensitivity at the time of sensing is improved.

As another example, when the resistance component SRa of the portion La of the graphene layer 130 and the resistance component SRc of the portion Lb of the graphene layer 130 are of high resistance, a disadvantage is caused in that not all the resistance components are reflected through channel modulation even if the resistance component SRb of the central portion Lb is converted from low resistance to high resistance.

Specifically, when the resistance component SRa of the portion La of the graphene layer 130 and the resistance component SRc of the portion Lb of the graphene layer 130 are 10 KΩ, respectively, and the resistance component SRb of the central portion Lb of the graphene layer 130 ranges from 1 to 10 KΩ, the total resistance of the graphene layer 130 is converted to a value between approximately 21KΩ and 30kΩ. Accordingly, the sensitivity at the time of sensing decreases significantly.

Thus, it is preferable that the lengths of the portion La of the graphene layer 130 and the portion Lb of the graphene layer 130 are kept to be less than 100 nm for the resistance component SRa of the portion La of the graphene layer 130 and the resistance component SRc of the portion Lb of the graphene layer 130 to have low resistance.

However, it can be difficult to implement a fabrication process that can form the lengths of the portion La of the graphene layer 130 and the portion Lb of the graphene layer 130 to be smaller than 100 nm.

Accordingly, in the present disclosure, at least a portion of the graphene layer 130 is separated from the substrate 110 so that the resistance component SRa of the portion La of the graphene layer 130 and the resistance component SRc of the portion Lb of the graphene layer 130 have low resistance. To this end, at least one concave pattern OM is formed on the substrate 110 or the insulating layer 120. For the structure above, refer to the description of the biosensor 100 of FIG. 1.

FIG. 4 is a drawing referenced to describe the biosensor according to one embodiment of the present disclosure of FIG. 1.

Referring to the figure, the biosensor 110a according to one embodiment of the present disclosure comprises a substrate 110; an insulating layer 120 on the substrate; a graphene layer 130, at least a portion of the lower surface of which is separated from the insulating layer 120, a source electrode 140a formed on one end of the graphene layer 130 and the substrate 110, a drain electrode 140b separated from the source electrode 140a and formed on the other end of the graphene layer 130 and the substrate 110, and a first passivation layer 160a and a second passivation layer 160b disposed on the source electrode 140a and the drain electrode 140b, respectively.

Meanwhile, the source electrode 140a can be formed on a first area PTa including one end of the graphene layer 130, the first passivation layer 160a can be formed on a second area PTb adjacent to the first area PTa of the graphene layer 130, a third area PTc adjacent to the second area PTb of the graphene layer 130 cannot be covered with the passivation layers 160a, 160b, the second passivation layer 160b can be formed on a fourth area PTd adjacent to the third area PTc of the graphene layer 130, and the drain electrode 140b can be formed on a fifth area PTe which includes the other end of the graphene layer 130 and is adjacent to the fourth area PTd.

In particular, since the area corresponding to the third area PTc in the lower surface of the graphene layer 130 is separated from the insulating layer 120, the sensing sensitivity of a graphene-based sensor can be improved.

The figure illustrates a case in which the source electrode 140a is formed on the first area PTa in the left part L1 of the graphene layer 130, and the drain electrode 140b is formed on the fifth area PTe in the right part L2 of the graphene layer 130.

The figure illustrates that the first passivation layer 160a is formed on the second area PTb of the graphene layer 130, the second passivation layer 160b is formed on the fourth area PTd, and the third area PTc of the graphene layer 130 is made open.

Meanwhile, since the first passivation layer 160a is formed on the second area PTb of the graphene layer 130, and the second passivation layer 160b is formed on the fourth area PTd, the length of the second area PTb and the length of the fourth area PTd are shorter than the lengths of La and Lb of FIG. 3, the resistance component of the second area PTb of the graphene layer 130 and the resistance component of the fourth area PTd exhibit a considerably low resistance value.

Accordingly, since the resistance component of the second area PTb of the graphene layer 130 and the resistance component of the fourth area PTd become small, all the resistance components are reflected through channel modulation even if the resistance component of the central area PTc of the graphene layer 130 is converted from low resistance to high resistance.

Specifically, when the resistance component of the second area PTb of the graphene layer 130 and the resistance component of the fourth area PTd are 1 KΩ, respectively, and the resistance component of the central area PTc of the graphene layer 130 ranges from 1 to 10 KΩ, the total resistance of the graphene layer 130 is converted to a value between approximately 3KΩ and 12kΩ. Accordingly, the sensitivity at the time of sensing is improved.

Meanwhile, it is preferable that the length of the second area PTb or the length of the fourth area PTd of the graphene layer 130 is shorter than the length of the third area PTc. Accordingly, since the resistance component of the second area PTb or the fourth area PTd of the graphene layer 130 decreases, the sensing sensitivity of a graphene-based sensor can be eventually improved.

Meanwhile, it is preferable that the length L4 of the second area PTb or the length L5 of the fourth area PTd of the graphene layer 130 is less than 100 nm. Accordingly, since the resistance component of the second area PTb or the fourth area PTd of the graphene layer 130 decreases, the sensing sensitivity of a graphene-based sensor can be eventually improved.

Meanwhile, it is preferable that the length of the second area PTb or the length of the fourth area PTd of the graphene layer 130 is shorter than the length of the first area PTa or the length of the fifth area PTe of the graphene layer 130. Accordingly, it is possible to improve the sensing sensitivity of a graphene-based sensor.

Meanwhile, as the resistance value of the second area PTb or the fourth area PTd of the graphene layer 130 decreases, the conductivity of the graphene layer 130 can increase. Accordingly, since the resistance component of the third area PTc of the graphene layer 130 acts as a primary factor, the sensing sensitivity of a graphene-based sensor can be improved eventually.

Meanwhile, as the length of the third area PTc of the graphene layer 130 increases, the conductivity of the graphene layer 130 can increase. Accordingly, since the resistance component of the third area PTc of the graphene layer 130 acts as a primary factor, the sensing sensitivity of a graphene-based sensor can be improved eventually.

Meanwhile, as the ratio of the length L4 of the second area PTb or the length L5 of the fourth area PTd of the graphene layer 130 to the length L3 of the third area PTc of the graphene layer 130 decreases, the conductivity of the graphene layer 130 can increase. Accordingly, since the resistance component of the second area PTb or the fourth area PTd of the graphene layer 130 decreases, the sensing sensitivity of a graphene-based sensor can be improved eventually.

Meanwhile, it is preferable that the height h3 of the third area PTc of the graphene layer 130 is smaller than the height h1 of the substrate 110 and smaller than the height h2 of the insulating layer 120.

Meanwhile, it is preferable that the height hk of the concave pattern OM is smaller than the height h4 of the source electrode 150a or the height h5 of the drain electrode 150b.

Meanwhile, the height hk of the concave pattern OM can be larger than the height h3 of the third area PTc of the graphene layer 130.

FIGS. 5 to 7B illustrate biosensors according to various embodiments of the present disclosure.

First, FIG. 5 shows one example of a side views of a biosensor according to another embodiment of the present disclosure.

Referring to the figure, the biosensor 100b according to another embodiment of the present disclosure is similar to the biosensor 100a of FIG. 4 but differs in that the third passivation layer Ata and the fourth passivation layer ATb are further formed on one end of the graphene layer 130 and on the lower part of the other end.

The biosensor 100b according to another embodiment of the present disclosure comprises a substrate 110; an insulating layer 120 on the substrate; a graphene layer 130 disposed on at least one concave pattern OM formed on the insulating layer 120, a source electrode 140a formed on one end of the graphene layer 130 and the substrate 110, a drain electrode 140b separated from the source electrode 140a and formed on the other end of the graphene layer 130 and the substrate 110, a first passivation layer 160a and a second passivation layer 160b disposed on the source electrode 140a and the drain electrode 140b, respectively, and a third passivation layer Ata and a fourth passivation layer ATb formed below one end and the other end of the graphene layer 130, respectively.

The first passivation layer 160a, the second passivation layer 160b, the third passivation layer ATa, and the fourth passivation layer ATb can be formed of the same material.

Meanwhile, the third passivation layer ATa and the fourth passivation layer ATb can be a part of the first passivation layer 160a and the second passivation layer 160b, respectively.

In other words, a portion ATa of the first passivation layer 160a can be formed below one end of the graphene layer 130, and a portion ATb of the second passivation layer 160b can be formed below the other end of the graphene layer 130. Accordingly, it is possible to improve the sensing sensitivity of a graphene-based sensor.

Meanwhile, since the area of the lower surface of the graphene layer 130 corresponding to the third area PTc is separated from the insulating layer 120, the sensing sensitivity of a graphene-based sensor can be improved.

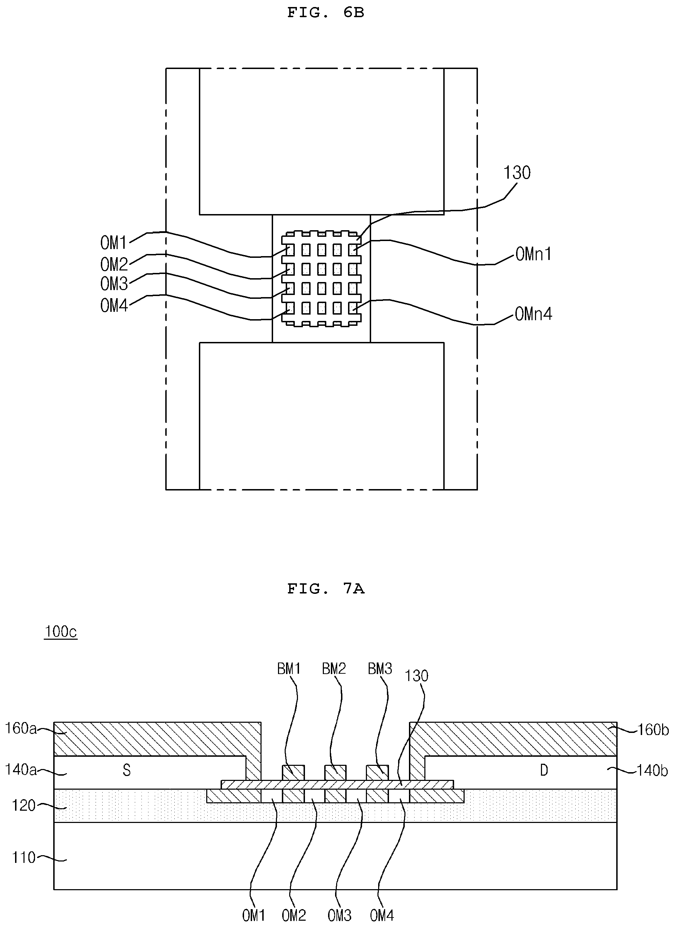

FIG. 6A is one example of a side view of a biosensor according to yet another embodiment of the present disclosure, and FIG. 6B is one example of a top view of the biosensor of FIG. 6A.

Referring to the figure, the biosensor 100c according to yet another embodiment of the present disclosure is similar to the biosensor 100b of FIG. 5 but differs in that a plurality of concave patterns OM1 to OM4 are formed instead of a single concave pattern.

The biosensor 100c according to yet another embodiment of the present disclosure comprises a substrate 110, an insulating layer 120 on the substrate, a graphene layer 130 disposed on a plurality of concave patterns OM1 to OM4 formed on the insulating layer 120, a source electrode 140a formed on one end of the graphene layer 130 and the substrate 110, a drain electrode 140b separated from the source electrode 140a and formed on the other end of the graphene layer 130 and the substrate 110, and a first passivation layer 160a and a second passivation layer 160b disposed on the source electrode 140a and the drain electrode 140b, respectively.

As described above, since the graphene layer is separated from the substrate 110 or the insulating layer 120 due to the plurality of concave patterns OM1 to OM4, the lattice mismatch between the graphene layer and the substrate can be reduced, thereby improving sensing sensitivity.

Meanwhile, a portion ATa of the first passivation layer 160a can be formed below one end of the graphene layer 130, and a portion ATb of the second passivation layer 160b can be formed below the other end of the graphene layer 130. Accordingly, it is possible to improve the sensing sensitivity of a graphene-based sensor.

According to the top view of FIG. 6B, the plurality of concave patterns OM1 to OMn4 within the biosensor 100c can be implemented in a mesh form; however, the patterns are not limited to the specific form and can also be implemented in various other forms such as hexagonal or dot patterns.

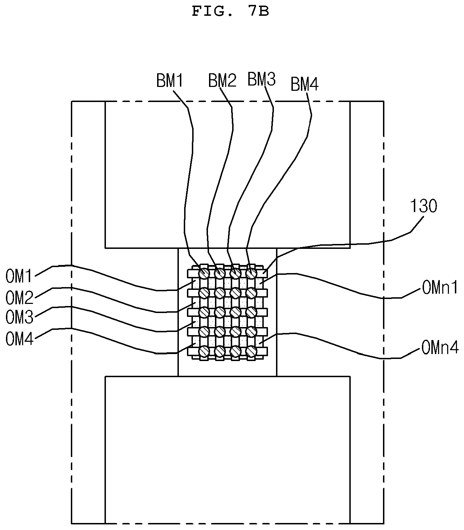

FIG. 7A is one example of a side view of a biosensor according to still another embodiment of the present disclosure, and FIG. 7B is one example of a top view of the biosensor of FIG. 7A.

Referring to the figure, the biosensor 100d according to still another embodiment of the present disclosure is similar to the biosensor 100c of FIG. 6 but differs in that a plurality of convex patterns BM1 to BM3 are formed on the graphene layer 130.

The biosensor 100d according to still another embodiment of the present disclosure comprises a substrate 110, an insulating layer 120 on the substrate, a graphene layer 130 disposed on a plurality of concave patterns OM1 to OM4 formed on the insulating layer 120, a source electrode 140a formed on one end of the graphene layer 130 and the substrate 110, a drain electrode 140b separated from the source electrode 140a and formed on the other end of the graphene layer 130 and the substrate 110, a first passivation layer 160a and a second passivation layer 160b disposed on the source electrode 140a and the drain electrode 140b, respectively, and a plurality of convex patterns BM1 to BM3 adjacent to the plurality of concave patterns OM1 to OM4 and formed on the graphene layer 130.

As described above, since the graphene layer is separated from the substrate 110 or the insulating layer 120 due to the plurality of concave patterns OM1 to OM4, the lattice mismatch between the graphene layer and the substrate can be reduced, thereby improving sensing sensitivity.

Meanwhile, since the contact area between the sample solution 180 and the upper surface of the graphene layer 130 increases due to the plurality of convex patterns BM1 to BM3, sensing sensitivity is improved.

Meanwhile, a portion ATa of the first passivation layer 160a can be formed below one end of the graphene layer 130, and a portion ATb of the second passivation layer 160b can be formed below the other end of the graphene layer 130. Accordingly, it is possible to improve the sensing sensitivity of a graphene-based sensor.

Meanwhile, according to FIG. 7A, the first passivation layer 160a, the second passivation layer 160b, the plurality of concave patterns OM1 to OM4, and the plurality of convex patterns BM1 to BM3 can all be formed of the same material.

Alternatively, unlike FIG. 7A, the plurality of concave patterns OM1 to OM4 can be formed of the same material as the insulating layer 120. On the other hand, the first passivation layer 160a, the second passivation layer 160b, and the plurality of convex patterns BM1 to BM3 can all be formed of the same material.

Alternatively, unlike FIG. 7A, the plurality of concave patterns OM1 to OM4 and the plurality of convex patterns BM1 to BM3 can be formed of the same material as the insulating layer 120.

According to the top view of FIG. 7B, the plurality of concave patterns OM1 to OMn4 within the bio sensor 100c can be implemented in a mesh form, and the plurality of convex patterns BM1 to BM3 can be formed in a mesh form adjacent to and between the plurality of concave patterns OM1 to OMn4; however, the patterns are not limited to the specific form and can also be implemented in various other forms such as hexagonal or dot patterns.

Throughout the document, preferred embodiments of the present disclosure have been described with reference to appended drawings; however, the present disclosure is not limited to the embodiments above. Rather, it should be noted that various modifications of the present disclosure can be made by those skilled in the art to which the present disclosure belongs without leaving the technical scope of the present disclosure defined by the appended claims, and these modifications should not be understood individually from the technical principles or perspectives of the present disclosure.

## Claims

1. A biosensor comprising:
a substrate;
a graphene layer disposed on at least one concave pattern formed on the substrate;
a source electrode formed on one end of the graphene layer and the substrate;
a drain electrode separated from the source electrode and formed on the other end of the graphene layer and the substrate; and
a first passivation layer and a second passivation layer respectively disposed on the source electrode and the drain electrode.

2. The biosensor of claim 1, wherein a portion of the first passivation layer is formed beneath one end of the graphene layer, and
a portion of the second passivation layer is formed beneath the other end of the graphene layer.

3. The biosensor of claim 1, wherein the graphene layer is disposed on a plurality of concave patterns formed on the substrate.

4. The biosensor of claim 1, wherein the graphene layer is disposed on a plurality of concave patterns formed on the substrate, and
a plurality of convex patterns are formed on the graphene layer, the plurality of convex patterns being adjacent to the plurality of concave patterns.

5. The biosensor of claim 4, wherein the plurality of convex patterns are formed of the same material as the first passivation layer and the second passivation layer.

6. The biosensor of claim 1, wherein the bio sensor further includes a gate electrode separated from the upper portion of either the first passivation layer or the second passivation layer.

7. The biosensor of claim 1, wherein a portion of the graphene layer is covered by the source electrode or the drain electrode and the first passivation layer or the second passivation layer; and
the other portion of the graphene layer is left open without being covered by the source electrode or the drain electrode and the first passivation layer or the second passivation layer.

8. The biosensor of claim 1, wherein the source electrode is formed on a first area which includes the one end of the graphene layer;
the first passivation layer is formed on a second area adjacent to the first area of the graphene layer;
the passivation layer is not covered on a third area adjacent to the second area of the graphene layer;
the second passivation layer is formed on a fourth area adjacent to the third area of the graphene layer; and
the drain electrode is formed on a fifth area which includes the other end of the graphene layer and is adjacent to the fourth area.

9. The biosensor of claim 8, wherein the length of the second area or the fourth area of the graphene layer is shorter than the length of the third area.

10. The biosensor of claim 8, wherein the length of the second area or the fourth area of the graphene layer is 100 nm or less.

11. The biosensor of claim 1, wherein the source electrode or the drain electrode includes an organic compound or an inorganic compound, and
the inorganic compound includes at least one of WO₃, MoO₃, or ZnO.

12. The biosensor of claim 1, further including an insulating layer disposed on the substrate,
wherein the source electrode, the drain electrode, and the graphene layer are disposed on the insulating layer.

13. A biosensor comprising:
a substrate;
a graphene layer formed on the substrate;
a source electrode formed on one end of the graphene layer and the substrate;
a drain electrode separated from the source electrode and formed on the other end of the graphene layer and the substrate; and
a first passivation layer and a second passivation layer disposed on the source electrode and the drain electrode, respectively,
wherein at least a portion of the lower surface of the graphene layer is separated from the substrate.

14. The biosensor of claim 13, wherein at least one concave pattern is formed in a partial area of the lower surface of the graphene layer within the substrate.

15. The biosensor of claim 13, wherein a plurality of concave patterns are formed in a partial area of the lower surface of the graphene layer within the substrate.

16. The biosensor of claim 13, wherein a plurality of concave patterns are formed in a partial area of the lower surface of the graphene layer within the substrate, and
a plurality of convex patterns are formed in a partial area of the upper surface of the graphene layer.
